# EUROPÄISCHE PATENTSCHRIFT

(11) **EP 0 539 839 B1**
(45) Veröffentlichungstag und Bekanntmachung des Hinweises auf die Patenterteilung: **14.01.1998**
(21) Anmeldenummer: 92117875.2
(22) Anmeldetag: 20.10.1992
(51) Int. Cl.: G01N 33/96, G01N 33/72

(54) **Stabiles flüssiges Kontroll- bzw. Eichserum für die klinische Diagnostik**
Stable liquid control- and calibration serum for clinical diagnostics
Serum stabilisée et liquide pour contrôller et calibrer pour la diagnostique clinique

(30) Priorität: 26.10.1991 DE 4135404
(43) Veröffentlichungstag der Anmeldung: 05.05.1993
(73) Patentinhaber: BOEHRINGER MANNHEIM GMBH, 68298 Mannheim (DE)
(72) Erfinder: Wild, Thomas, Dr., W-8120 Weilheim (DE); Stegmüller, Peter, W-8900 Augsburg (DE)

(56) Entgegenhaltungen:
- EP-A- 0 082 587
- EP-A- 0 131 826
- EP-A- 0 184 765
- US-A- 3 876 375
- US-A- 4 344 864
- EXPERIENTIA Bd. 28, Nr. 4, 15. April 1972, BASEL CH Seiten 379 - 380 P. MANITTO UND D. MONTI 'Photoaddition of Sulphydryl Groups to Bilirubin in vitro'
- DATABASE WPIL Section Ch, Week 8647, Derwent Publications Ltd., London, GB; Class B04, AN 86-308037

## Beschreibung

Gegenstand der Erfindung ist ein stabiles flüssiges Kontrollbzw. Eichserum für die klinische Diagnostik, das ein flüssigstabiles Bilirubinderivat enthält.

Zur Richtigkeits- und Präzisionskontrolle von Bestimmungsmethoden und zum Kalibrieren von Analysenautomaten werden üblicherweise Kontroll- oder Eichseren oder als Kontroll- bzw. Eichserum genannte Zusammensetzungen eingesetzt, die die zu bestimmenden Parameter in bekannten, genau festgelegten Konzentrationen enthalten. Man unterscheidet Spezialkontroll- bzw. Eichseren, die zur Kontrolle bzw. zur Eichung von ganz bestimmten Parametern oder einer begrenzten, meist zusammengehörigen Gruppe von Parametern dienen und sogenannte Universalkontroll-bzw. Eichseren, die zur Kontrolle bzw. zum Eichen möglichst aller gängigen Parameter nach sämtlichen in der Praxis üblicherweise durchgeführten Bestimmungsmethoden einsetzbar sind.

Die Anforderungen, die an solche Produkte im klinisch/ chemischen Routinebetrieb gestellt werden, umfassen:
- Eine genau bekannte Konzentration der Meßparameter muß eingehalten werden.
- Die Konzentrationen müssen im medizinisch relevanten Meßbereich (normal bzw. pathologisch) liegen.
- Die Handhabung der Kontroll- bzw. Eichseren muß einfach sein.
- Die Kontroll- bzw. Eichseren müssen eine möglichst lange Haltbarkeit besitzen.

Bei einem Einsatz des Kontroll- bzw. Eichserums bei Analysenautomaten werden hohe Anforderungen besonders an die beiden letzten Punkte, Handhabung und Haltbarkeit, gestellt. Bisher werden Kontroll- bzw. Eichseren meist in lyophilisierter Form angeboten, wodurch eine akzeptable Haltbarkeit vor der Rekonstitution gewährleistet wird. Diese Methode ist aber aufwendig und verlangt vor Gebrauch eine Rekonstitution mit einer Rekonstitutionsflüssigkeit. Lyophilisierte Produkte neigen danach oft zur Trübungsbildung, wodurch Fehler in der klinischen Diagnostik auftreten können, da viele Nachweismethoden kolorimetrische Auswertungen verlangen. Auch können Fehler durch ungenaues Pipettieren der Rekonstitutionsflüssigkeit eingeschleppt werden. Nach der Rekonstitution sind diese Kontrollbzw. Eichseren meist nur einige Stunden bei Raumtemperatur haltbar, so daß sie täglich neu hergestellt werden müssen. Für eine einfache Handhabung ist es daher wünschenswert, ein flüssiges Kontroll- bzw. Eichserum zur Verfügung zu stellen, das ohne weitere Aufarbeitung durch das Bedienungspersonal der Analysenautomaten nutzbar ist.

In der US 3,876,375 wird eine flüssige biologische Zusammensetzung, die als Referenzkontrolle bei diagnostischen Analysen eingesetzt werden kann, beschrieben. Zur Stabilisierung werden 20 - 40 % Alkylenpolyole mit 2 - 5 C-Atomen, wie Ethylenglycol, Propylenglycol, Butylenglycol, Pentandiol und Glycerin zugesetzt. Das Kontroll- bzw. Eichserum kann durch die Zugabe derart hoher Konzentrationen an Polyolen viskos werden. Dies kann bei den Pump- und Pipettierschritten der automatischen Analyse zu Schwierigkeiten und Ungenauigkeiten führen, weshalb die Zugabe hoher Konzentrationen an Alkylenpolyolen nicht zweckmäßig ist.

Bei der Herstellung eines flüssigstabilen Kontroll- bzw. Eichserums ist das Substrat Bilirubin meist der stabilitätslimitierende Faktor. Der Bilirubingehalt wird bei der Diagnose der Hyperbilirubinämie bestimmt. Im lyophilisierten Zustand ist Bilirubin ausreichend stabil, während nach der Rekonstitution die Haltbarkeit bei Zimmertemperatur nur wenige Stunden, bei -20 °C ca. zwei Wochen beträgt.

In US 4,344,864 wird die Stabilisierung von konjugiertem Bilirubin durch Zusatz einer Sulfhydrylkomponente und eines Chelators bei einem pH von 8,2 - 9,2 in Blutserum beschrieben. Bei der Herstellung von Universalkontroll- bzw. Eichseren ist es allerdings vorteilhaft, so wenig wie möglich Zusatzstoffe zu verwenden. Ein Stabilisator für einen bestimmten Parameter kann sich oft bei der Bestimmung anderer Parameter störend auswirken. Reduzierende SH-Verbindungen können z. B. bei der anschließenden enzymatischen Umsetzung stören.

Aufgabe war es daher, ein flüssiges Kontroll- bzw. Eichserum bereitzustellen, das bei der Bestimmung von mehreren Parametern der klinischen Diagnostik eingesetzt werden kann und Bilirubin in einer stabilisierten Form enthält. Die Aufgabe wird durch die in den Ansprüchen näher charakterisierte Erfindung gelöst. Im wesentlichen wird die Aufgabe dadurch gelöst, daß das Kontroll- bzw. Eichserum ein flüssigstabiles Bilirubinderivat enthält.

Gegenstand der Erfindung ist somit ein stabiles flüssiges Kontroll- bzw. Eichserum für die klinische Diagnostik, das ein flüssigstabiles Bilirubinderivat enthält.

Weiterer Gegenstand der Erfindung ist ein Verfahren zur Herstellung eines stabilen flüssigen Kontroll- bzw. Eichserums für die klinische Diagnostik, das ein flüssigstabiles Bilirubinderivat enthält, ausgehend von einer Rinderserumalbuminlösung, die mit den gewünschten Parametern bis zur gewünschten Parameterkonzentration vermischt wird, wobei gegebenenfalls Hilfsmittel zugesetzt werden.

Weiterer Gegenstand der Erfindung ist die Verwendung eines flüssigstabilen Bilirubinderivates in einem Kontroll- bzw. Eichserum. Das Bilirubinderivat dient zur Erhöhung der Lagerstabilität des Kontroll- bzw. Eichserums.

Das erfindungsgemäße stabile flüssige Kontroll- bzw. Eichserum enthält für die klinische Diagnostik wichtige Analytparameter. Bicarbonat, Bilirubin, Cholesterin, Creatinin, Eisen, Glucose, Harnsäure, Harnstoff, Lactat, Triglyceride, Phospholipide, Gesamtprotein, Albumin, Calcium, Phosphat, Magnesium, Natrium, Kalium und Chlorid können als Parameter vorkommen. Gegebenenfalls können noch klinisch relevante Enzyme enthalten sein.

Die Parameterkonzentrationen liegen vorzugsweise im klinischen Entscheidungsbereich, d. h. in Konzentrationsbereichen, die in normalen oder pathologischen Patientenseren auftreten. Die Konzentrationen der einzelnen Parameter können selbstverständlich von Charge zu Charge in den in der Tabelle 1 angegebenen Grenzen variieren.

**Tabelle 1**

| Konzentrationsbereiche für die einzelnen Parameter eines flüssigen Kontroll- bzw. Eichserums | |
|---|---|
| Substanz | Konzentrationsbereich [mg/dl] |
| Bicarbonat | 268 - 317 |
| Bilirubin | 0,25 - 6,00 |
| Cholesterin | 125 - 200 |
| Creatinin | 0,5 - 3,0 |
| Eisen | 0,06 - 0,3 |
| Glucose | 70 - 210 |
| Harnsäure | 2,5 - 8,5 |
| Harnstoff | 10 - 150 |
| Lactat | 6 - 22 |
| Triglyceride | 70 - 210 |
| Phospholipide | 150 - 280 |
| Gesamtprotein | 5000 - 9000 |
| Albumin | 3500 - 7000 |
| Calcium | 9 - 14 |
| Phosphat | 2,5 - 7 |
| Magnesium | 1,8 - 4,5 |
| Natrium | 260 - 360 |
| Kalium | 14 - 22,5 |
| Chlorid | 280 - 385 |

Das erfindungsgemäße stabile flüssige Kontroll- bzw. Eichserum enthält als Parameter für die Bilirubinbestimmung nicht freies Bilirubin selbst, sondern ein Bilirubinderivat. Die erfindungsgemäßen Bilirubinderivate zeigten überraschenderweise eine erheblich höhere Stabilität bei flüssiger Aufbewahrung als underivatisiertes Bilirubin. Als Ausgangsverbindung zur Herstellung der Bilirubinderivate können freies Bilirubin, dessen lösliche Alkalisalze, z. B. Natrium-Bilirubinat, Bilirubinester oder Bilirubinamide verwendet werden. Wesentlich für die Stabilisierung der Bilirubinderivate ist, daß an die in Position 18 am Tetrapyrrolring vorhandene Vinylgruppe ein Alkohol oder ein Mercaptan, d. h. eine Thiolgruppe enthaltende Verbindung, addiert wird. Beispielsweise können Thioglykolsäure, Methylthioglykolsäure, 2-Mercaptoethanol oder N-Acetyl-L-Cystein addiert werden. Solche Bilirubinderivate sind aus Experientia 28 (1972), 379 - 380 bekannt. Die erfindungsgemäß im Kontrollbzw. Eichserum einsetzbaren Bilirubinderivate werden durch die folgende allgemeine Formel (I) wiedergegeben: 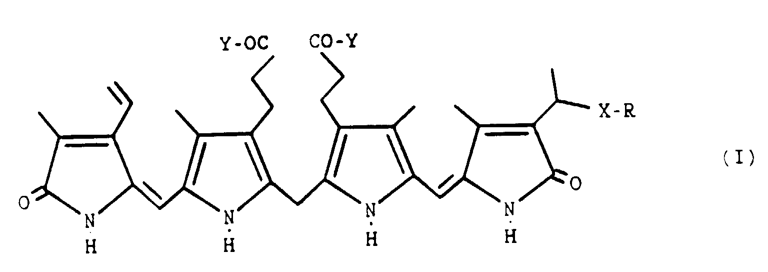 wobei
- X: Sauerstoff oder Schwefel,
- R: eine ein- oder mehrfach durch Carboxy, Alkoxycarbonyl, Hydroxy, Mercapto, Amino, Alkylamino, Dialkylamino oder Alkylcarbonylamino substituierte C₁-C₆-Alkylgruppe und
- Y: Amino, Alkylamino, Dialkylamino, Hydroxy, Alkoxy oder deren Alkalisalze bedeuten.

Ganz besonders bevorzugt wird Bilirubinthioglycolsäureether eingesetzt.

Das erfindungsgemäße flüssige Kontroll- bzw. Eichserum kann neben den Analytparametern noch zusätzliche Hilfsstoffe, beispielsweise weitere Analytstabilisatoren, Detergenzien und Konservierungsstoffe enthalten. Dabei ist bei dem Zusatz dieser Hilfsstoffe stets darauf zu achten, daß die Messung bestimmter Parameter nicht störend beeinflußt wird. Die Zugabe von Ascorbinsäure bevorzugt in Konzentrationen von 0,5 bis 1,5 mg/dl, besonders bevorzugt 0,9 mg/dl, führt beispielsweise zu einer weiteren Steigerung der Stabilität der Bilirubinderivate. Durch eine Aufbewahrung des Reagenzes unter einer Schutzgasatmosphäre, besonders einer N₂-Schutzgasatmosphäre, in Kombination mit dem Ascorbinsäurezusatz, wird eine besonders hohe Stabilität des Bilirubinderivats erzielt. Die Schutzgasatmosphäre wirkt sich außer auf die Stabilität des Bilirubinderivats sehr günstig auf die Stabilität anderer Parameter, wie z. B. Harnsäure aus. Die Stabilisierung des Bicarbonatwertes wird durch Zusatz von ca. 0,3 Vol.-% CO₂ zum N₂-Inertgas erreicht. Neben diesen Analystabilisatoren können noch weitere einzelne Analytstabilisierende Hilfsstoffe zugesetzt werden. So wirkt sich zum Beispiel ein Zusatz von EDTA stabilisierend auf den Eisenwert aus. Die Zugabe einer verdünnten Fe-Lösung bei der Herstellung des Kontroll- bzw. Eichserums in Gegenwart von EDTA, bevorzugt in einem Verhältnis von 1:1,1 (Fe/EDTA), führt zu stabilisierten Fe-Komplexen, die nicht von Lipoproteinen bzw. Rinderserumalbumin zerstört werden.

Die Zugabe von Creatin wirkt sich positiv auf die Stabilität des Parameters Creatinin aus, da die Creatinin-Hydrolyse hierdurch verhindert oder zumindest stark vermindert wird.

Für den Triglycerid-Parameter wird in bisher gebräuchlichen Kontroll- bzw. Eichseren eine Eigelbfraktion eingesetzt. Trübungen sind bei einer solchen Zusammensetzung nicht auszuschließen. Durch den Ersatz dieser Eigelbfraktion durch Glycerin als Triglycerid kann diese potentielle Störung gänzlich vermieden werden. Glycerin zeigt auch bei längerer Lagerung keine Neigung zur Trübungsbildung und ist daher für den Einsatz im erfindungsgemäßen flüssigen Kontroll- bzw. Eichserum vorzüglich geeignet.

Als Konservierungsstoffe sind prinzipiell bekannte Konservierungsstoffe zu verwenden. Es muß allerdings darauf geachtet werden, daß die Messung der Parameter nicht negativ beeinflußt wird. Vorteilhaft für die Konservierung des erfindungsgemäßen flüssigen Kontroll- bzw. Eichserums hat sich eine Kombination von fungiziden und bakteriziden Konservierungsmitteln erwiesen. Besonders bevorzugt ist die Verwendung von Gentamycin in einer Konzentration von 100 - 150 µg/ml in Kombination mit 2,4-Dichlorbenzylalkohol in einer Konzentration von 150 - 250 mg/dl. Die Verwendung einer Schutzgasatmosphäre besitzt weiterhin einen positiven Effekt auf die Konservierung.

Der pH-Wert des flüssigen Kontroll- bzw. Eichserums wird durch Verwendung eines Puffers auf einen schwach alkalischen Wert eingestellt, bevorzugt auf einen pH von 8,2 bis 8,4. Die Verwendung von 20 -100 mM TAPS-Puffer, insbesondere 50 mM, pH 8,3, hat sich günstig erwiesen. Der leicht alkalische pH wirkt sich positiv auf die Stabilität des Kontroll- bzw. Eichserums aus, da ein Pilzwachstum gehemmt wird. Hierdurch ist es möglich, die Konzentration des Fungizids - falls dieser Hilfsstoff erforderlich erscheint - gering zu halten und Störungen bei der Bestimmung der Parameter auszuschließen.

Zur Herstellung des erfindungsgemäßen stabilen flüssigen Kontroll- bzw. Eichserums geht man bevorzugt von einer Serumalbuminlösung aus. Besonders bevorzugt wird eine Rinderserumalbuminlösung anstelle des bisher üblichen Humanserums eingesetzt. Die Rinderserumalbuminlösung zeigt den Vorteil, daß sie nicht infektiös ist, vor allem im Hinblick auf Infektionen mit Hepatitis- und HIV-Viren. Weiterhin ermöglicht die Verwendung einer Rinderserumalbuminlösung im Vergleich zum Humanserum eine genau definierte und reproduzierbare Grundrezeptur mit wesentlich geringeren Chargenschwankungen. In dieser Grundmatrix wird in üblicher Weise die gewünschte Parameterkonzentration eingestellt.

Zur Herstellung kann ebenfalls von einem trockenen Gemisch, das die gewünschten Analytparameter einschließlich des Bilirubinderivats der Formel (I) enthält, ausgegangen werden. Dieses Gemisch wird bis zur gewünschten Analytparameterkonzentration in Wasser gelöst.

Das erfindungsgemäße flüssige Kontroll- bzw. Eichserum ist im ungeöffneten Zustand bei 4 °C über 12 Monate hinaus stabil. Durch eine Aufbewahrung bei -20 °C kann diese Stabilität noch deutlich erhöht werden. Auch in geöffnetem Zustand bei 10 °C ist das Produkt mehrere Tage haltbar. Es eignet sich hervorragend als Qualitätskontroll- und Eichserum für Parameter der klinischen Diagnostik, insbesondere unter Berücksichtigung von Bilirubin. Besonders bevorzugt wird das erfindungsgemäße flüssige Kontroll- bzw. Eichserum bei der automatischen klinischen Diagnostik verwendet.

Die Erfindung wird durch folgende Beispiele näher erläutert:

### Beispiel 1

### Synthese von Bilirubinthioglycolsäureether

Eine sauerstofffreie Lösung aus 10 g Bilirubin und 156 g Thioglycolsäure in 6 l Dimethylformamid und 30 ml Triethylamin bestrahlt man unter Stickstoffatmosphäre 20 Stunden mit einer Quecksilberdampflampe bei einer Wellenlänge > 300 nm. Die Temperatur der Lösung soll dabei zwischen 30 und 60 °C liegen. Die Umsetzung läßt sich dünnschichtchromatographisch verfolgen (DC: Kieselgel 60, Eluens Toluol/Methanol/Essigsäure = 87/9/4). Nach Abschluß der Reaktion wird Dimethylformamid und Triethylamin im Hochvakuum abgezogen. Das Produkt fällt dabei aus der höher siedenden Thioglykolsäure aus und wird abfiltriert. Die verbleibende Lösung versetzt man mit ca. 150 ml Wasser, um Reste des Produktes auszufällen. Aus den vereinigten Filtraten wäscht man mit 500 ml Wasser und anschließend mit 200 ml kaltem Methanol restliche Spuren der Thioglykolsäure heraus. Man erhält nach Trocknen im Hochvakuum bei 30 °C 8 g (69 %) rotes Produkt.
DC (Kieselgel 60, Eluens Toluol/Methanol/Essigsäure = 87/9/4), RF = 0,3
MS (negativ FAB): m/e = 675 (M-H).

### Beispiel 2

### Herstellung des flüssigen Kontroll- bzw. Eichserums für die klinische Diagnostik

Für einen 100 1-Ansatz werden 60 l Wasser mit 3,718 g EDTA Natriumsalz, 1,194 g FeSO₄ x 7 H₂O, ca. 10 l Lipoproteinfraktion, 241 ml Gentamycinsulfat-Lösung, 200 g Myacide® SP (2,4-Dichlor-benzylalkohol) und 6 kg Rinderserumalbumin vermischt. Dieser erste Ansatz wird über Nacht bei 4 °C gerührt, wodurch Rinderserumalbumin und Myacide® SP in Lösung gehen.

Am zweiten Tag werden folgende Substanzen, die vorher gelöst wurden, zugegeben:
37,28 g KCl in 200 ml H₂ O gelöst
44,11 g CaCl₂ x 2 H₂ O in 100 ml H₂ O gelöst
30,50 g MgCl₂ x 6 H₂ O in 100 ml H₂ O gelöst
22,65 g Na-L-Lactat in 100 ml H₂ O gelöst
26,73 g NaH₂ PO₄ x H₂ O in 100 ml H₂ O gelöst
198 g Glucose x H₂ O in 1,0 l H₂ O gelöst
130 g Harnstoff in 1,0 l H₂ O gelöst
2,2 g Creatinin in 100 ml H₂ O gelöst
7,7 g Creatin x H₂ O in 100 ml H₂ O gelöst
ca. 22 g Glycerin in 100 ml H₂ O gelöst
7,0 g Li₂ CO₃ + 7,0 g Harnsäure in 1,0 l H₂ O gelöst
1217 g TAPS in 2,5 l H₂ O gelöst
881 mg Ascorbinsäure in 100 ml H₂ O gelöst
318 g Na₂ CO₃ in 3,0 l H₂ O gelöst
6,6 g Bilirubinthioglycolsäureether in Na₂ CO₃-Lösung gelöst

200 - 400 g NaCl (ad 135 mM Na⁺) in 2,0 l H₂ O gelöst
ca. 120 g Tetramethylammoniumchlorid (ad 95 mM Cl⁻) in 1,0 l H₂ O gelöst
400 - 500 ml 2 N Essigsäure (oder u.U. 2 N NaOH) ad pH = 8,3 H₂ O ad 100 l

Das flüssige Kontroll- bzw. Eichserum wird anschließend steril filtriert und in geeignete Gefäße abgefüllt. Über Nacht erfolgt eine Stickstoffbegasung, wonach die Gefäße verschlossen werden.

Das so hergestellte flüssige Kontroll- bzw. Eichserum enthält die folgenden Bestandteile in den in der Tabelle 2 angegebenen Konzentrationen.

**Tabelle 2**

| Analytkonzentrationen im flüssigen Kontroll- bzw. Eichserum | |
|---|---|
| Analyt | Konzentration |
| Bilirubin | 4,8 mg/dl |
| Cholesterin | 150 mg/dl |
| Creatinin | 2,2 mg/dl |
| Eisen | 240 µg/dl |
| Glucose | 180 mg/dl |
| Harnsäure | 7,0 mg/dl |
| Harnstoff | 130 mg/dl |
| Lactat | 18 mg/dl |
| Triglyceride | 180 mg/dl |
| Phospholipide | 195 mg/dl |
| Gesamtprotein | 6,0 g/dl |
| Albumin | 6,0 g/dl |
| Calcium | 3,0 mmol/l |
| Phosphat | 6,0 mg/dl |
| Magnesium | 1,5 mmol/l |
| Natrium | 135 mmol/l |
| Kalium | 5,0 mmol/l |
| Chlorid | 95 mmol/l |

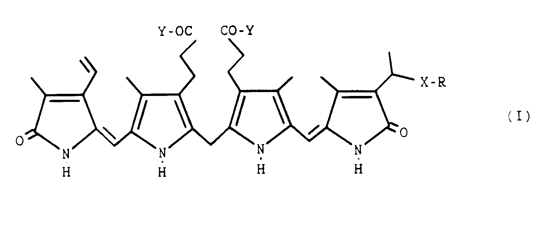

## Patentansprüche

1. Stabiles flüssiges Kontroll- bzw. Eichserum für die klinische Diagnostik, das ein flüssigstabiles Bilirubinderivat der allgemeinen Formel (I) 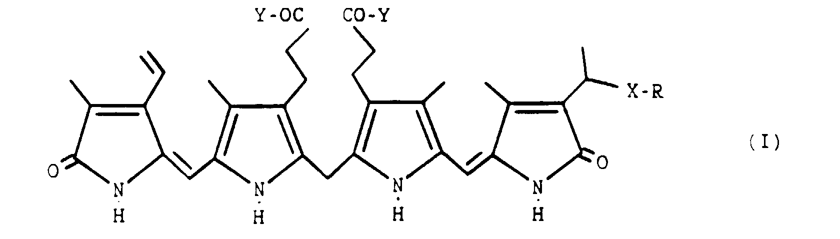 enthält, wobei
X Sauerstoff oder Schwefel,
R eine ein- oder mehrfach durch Carboxy, Alkoxycarbonyl, Hydroxy, Mercapto, Amino, Alkylamino, Dialkylamino oder Alkylcarbonylamino substituierte C₁-C₆-Alkylqruppe und
Y Amino, Alkylamino, Dialkylamino, Hydroxy, Alkoxy oder deren Alkalisalze bedeuten.

2. Ein Kontroll- bzw. Eichserum gemäß Anspruch 1, das das flüssigstabile Bilirubinderivat Bilirubinthioglycolsäureether enthält.

3. Ein Kontroll- bzw. Eichserum gemäß einem der Ansprüche 1 und 2, das weitere Konservierungsmittel und Analytstabilisatoren enthält.

4. Ein Kontroll- bzw. Eichserum gemäß Anspruch 3, das als Analytstabilisator Ascorbinsäure enthält und unter Schutzgas aufbewahrt wird.

5. Ein Kontroll- bzw. Eichserum gemäß einem der Ansprüche 3 und 4, in dem als Konservierungsmittel eine Kombination aus bakteriziden und fungiziden Substanzen eingesetzt wird.

6. Ein Kontroll- bzw. Eichserum gemäß Anspruch 5, das Gentamycin und 2,4-Dichlorbenzylalkohol enthält.

7. Ein Kontroll- bzw. Eichserum gemäß einem der Ansprüche 5 und 6 mit einem pH von 8,2 - 8,6.

8. Ein Kontroll- bzw. Eichserum gemäß einem der Ansprüche 1 - 7, das den Analytstabilisator Creatin enthält.

9. Ein Kontroll- bzw. Eichserum gemäß einem der Ansprüche 1 - 8, das auf Rinderserumalbuminbasis zusammengesetzt ist.

10. Verfahren zur Herstellung eines Kontroll- bzw. Eichserums gemäß einem der Ansprüche 1 - 9, dadurch gekennzeichnet, daß man eine Rinderserumalbuminlösung mit den gewünschten Analytparametern bis zur gewünschten AnalytparameterKonzentration vermischt und gegebenenfalls Hilfsmittel zusetzt.

11. Verfahren zur Herstellung eines Kontroll- bzw. Eichserums gemäß einem der Ansprüche 1 - 9, dadurch gekennzeichnet, daß man ein trockenes Gemisch, das die Analytparameter einschließlich des Bilirubinderivats der Formel (I) enthält, bis zur gewünschten Analytparameterkonzentration in Wasser auflöst.

12. Verwendung des Bilirubinderivats der Formel (I) in einem Kontroll- bzw. Eichserum.

13. Verwendung des Bilirubinderivats der Formel (I) zur Erhöhung der Lagerstabilität von Kontroll- bzw. Eichseren.

## Claims

1. Stable liquid control and/or calibration serum for use in clinical diagnostics, said serum containing a liquid-stable bilirubin derivative of the general formula (I) 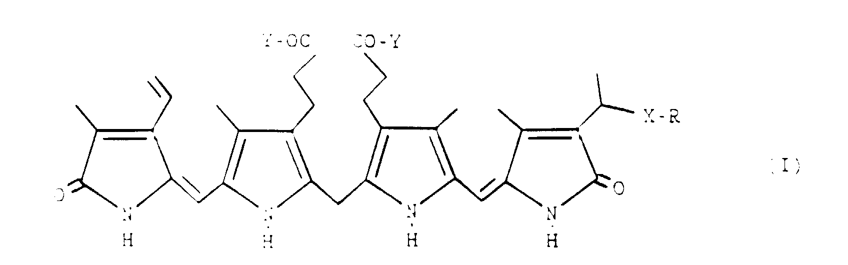 wherein
X stands for oxygen or sulfur
R stands for a C₁-C₆-alkyl group which is mono- or poly-substituted with carboxy, alkoxy carbonyl, hydroxy, mercapto, amino, alklyamino, dialkylamino or alkylcarbonylamino and
Y stands for amino, alkylamino, dialkylamino, hydroxy, alkoxy groups, or the alkali salts thereof.

2. Control and/or calibration serum according to claim 1 containing the liquid-stable bilirubin derivative bilirubin thioglycolic acid ether.

3. Control and/or calibration serum according to one of the claims I and 2 containing additional preservatives and analyte stabilizers.

4. Control and/or calibration serum according to claim 3 containing ascorbic acid as an analyte stabilizer and stored under protective gas.

5. Control and/or calibration serum according to one of the claims 3 and 4 in which a combination of bactericidal and fungicidal substances are used as a preservative.

6. Control and/or calibration serum according to claim 5 containing gentamycin and 2,4-dichlorobenzyl alcohol.

7. Control and/or calibration serum according to one of the claims 5 and 6 having a pH of 8.2 - 8.6.

8. Control and/or calibration serum according to one of the ciaims 1 - 7 containing the analyte stabilizer creatine.

9. Control and.or calibration serum according to one of the claims 1 - 8 whose composition is based on bovine serum albumin.

10. Process for the preparation of a control andior calibration serum according to one of the claims 1 - 9, charactenzed in that a bovine serum albumin solution is mixed with the desired analyte parameters until the desired concentration of the analyte parameters is obtained and that additives are added, if necessary.

11. Process for the preparation of a control and/or calibration serum according to one of the claims 1 - 9, characterized in that a dry mixture, which contains the analyte parameter including the bilirubin derivative of the formula (I), is dissolved in water until the desired concentration of the analyte parameters is obtained.

12. Use of the bilirubin derivative of the formula (I) in a control and/or calibration serum.

13. Use of the bilirubin derivative of the formula (I) to increase the storage stability of control and/or calibration sera.

## Revendications

1. Sérum témoin ou étalon liquide, stable, destiné au diagnostic clinique, qui contient un dérivé de la bilirubine stable vis-à-vis des liquides, de formule générale I 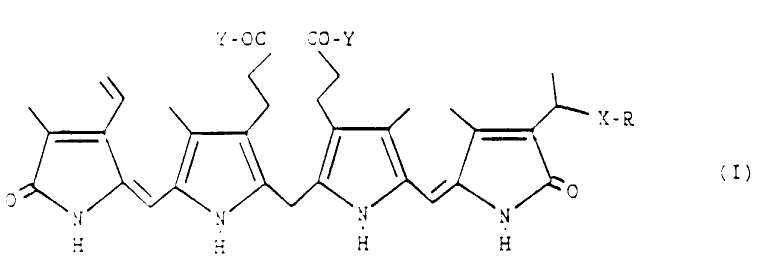 où
X représente un atome d'oxygène ou de soufre,
R représente un groupe alkyle en C₁-C₆ substitué une ou plusieurs fois par un groupe carboxy, alcoxycarbonyle, hydroxy, mercapto, amino, alkylamino, dialkylamino ou alkylcarbonyl-amino, et
Y représente un groupe amino, alkylamino, dialkylamino, hydroxy, alcoxy ou leurs sels de métaux alcalins.

2. Sérum témoin ou étalon selon la revendication 1, qui contient, en tant que dérivé de la bilirubine stable vis-à-vis des liquides, de l'éther de l'acide bilirubinethioglycolique,.

3. Sérum témoin ou étalon selon la revendication 1 ou 2, qui contient en outre un conservateur et des stabilisants d'analyte.

4. Sérum témoin ou étalon selon la revendication 3, qui contient, en tant que stabilisant d'analyte, de l'acide ascorbique et qui est conservé sous gaz de protection.

5. Sérum témoin ou étalon selon l'une des revendications 3 et 4, dans lequel, en tant que conservateur, on utilise une combinaison de substances bactéricides et fongicides.

6. Sérum témoin ou étalon selon la revendication 5, qui contient de la gentamycine et de l'alcool 2,4-dichlorobenzylique.

7. Sérum témoin ou étalon selon l'une des revendications 5 et 6, ayant un pH de 8.2-8.6.

8. Sérum témoin ou étalon selon l'une quelconque des revendications 1 à 7, qui contient de la créatine en tant que stabilisant d'analyte.

9. Sérum témoin ou étalon selon l'une quelconque des revendications 1 à 8, dont la composition est à base d'albumine de sérum bovin.

10. Procédé de préparation d'un sérum témoin ou étalon selon l'une quelconque des revendications 1-9, caractérisé en ce que l'on mélange une solution d'albumine de sérum bovin avec les paramètres d'analyte souhaités jusqu'à obtention d'une concentration de paramètres d'analyte souhaitée, et éventuellement, on ajoute un adjuvant.

11. Procédé de préparation d'un sérum témoin ou étalon selon l'une quelconque des revendications 1-9, caractérisé en ce que l'on dissout dans de l'eau un mélange sec qui contient les paramètres d'analyte, y compris le dérivé de la bilirubine de formule (I), jusqu'à obtention d'une concentration de paramètres d'analyte souhaitée.

12. Utilisation du dérivé de la bilirubine de formule (I) dans un sérum témoin ou étalon.

13. Utilisation du dérivé de la bilirubine de formule (I) pour augmenter la stabilité au stockage des sérums témoins ou étalons.
